# EUROPEAN PATENT APPLICATION

(11) **EP 4 732 926 A1**
(43) Date of publication of application: **29.04.2026**
(21) Application number: 24208978.7
(22) Date of filing: 25.10.2024
(51) Int. Cl.: B01D 15/16, B01D 15/32, B01J 20/287, C07K 1/20, B01D 15/12

(54) **MULTIVALENT HALIDE SALTS FOR USE IN HYDROPHOBIC INTERACTION CHROMATOGRAPHY**

(71) Applicant: Sartorius BIA Separations d.o.o., 5270 Ajdovscina (SI)
(72) Inventor: Sekirnik, Rok, 5270 Ajdovscina (SI); Bo ic, Klemen, 5270 Ajdovscina (SI); Sedlar, Ajda, 5270 Ajdovscina (SI)
(74) Representative: Cohausz & Florack

(57) **Abstract**

The present invention relates to the use of multivalent halide salts in hydrophobic interaction chromatography. The present invention further relates to a method for purifying biomolecules using hydrophobic interaction chromatography with multivalent halide salts. The present invention further relates to a use of a chromatography support in a method according to the invention. The present invention further relates to a Kit for the purification of biomolecules comprising multivalent halide salts.

## Description

The present invention relates to the use of multivalent halide salts in hydrophobic interaction chromatography. The present invention further relates to a method for purifying biomolecules using hydrophobic interaction chromatography with multivalent halide salts. The present invention further relates to a use of a chromatography support in a method according to the invention. The present invention further relates to a Kit for the purification of biomolecules comprising multivalent halide salts.

It is known to use kosmotropic salts as mobile phase modifier for hydrophobic interaction chromatography. Kosmotropic salts are thought of as a type of salt that stabilizes water structure and increases the ordering of water molecules around hydrophobic surfaces. These salts have a strong ability to promote hydrophobic interactions and stabilize macromolecules like proteins, often by increasing the strength of hydrogen bonds within the water network. Kosmotropic salts include, but are not limited to, ammonium sulfate, sodium sulfate, potassium phosphate, sodium citrate, and potassium citrate. In hydrophobic interaction chromatography, kosmotropic salts are often used in the mobile phase to enhance the hydrophobic interactions between the biomolecules and the stationary phase, promoting their binding to the column. As the salt concentration is reduced, the hydrophobic interactions weaken, leading to the elution of the bound molecules. A commonly used mobile phase modifier in hydrophobic interaction chromatography is ammonium sulfate.

However, the use of ammonium sulfate poses serious challenges due to the difficulties associated with the disposal of ammonium sulfate. When ammonium sulfate enters natural bodies of water, it leads to eutrophication of the water ecosystem, promoting unwanted growth of algae and other organisms which may lead to a disruption of the ecosystem. Use of ammonium sulfate may therefore be associated with significant environmental issues, and there is a need in the art for environmentally friendly alternatives.

Against this background, it is an object of the present invention to provide a mobile phase modifier for hydrophobic interaction chromatography applications that overcomes the disadvantages lined out above while maintaining satisfactory functionality and quality of the chromatography results achieved.

The object named above is solved in accordance with the present invention by a use of at least one multivalent halide salt as mobile phase modifier in hydrophobic interaction chromatography.

In the context of the present invention, the term "halide" is intended to be understood as a compound comprising a halogen in form of a negatively charged ion (anion). Halogens include fluorine (F), chlorine (Cl), bromine (Br), iodine (I), and astatine (At). Halides therefore comprise one or more of the anions F-, CF, Br, I-, and At.

In the context of the present invention, the term "salt" is intended to be understood as an ionic compound consisting of positively charged cations and negatively charged anions that are held together by ionic bonds. A salt can be present in complexed form, e.g. in form of a hydrate, or can be water-free. In the context of the present invention, a "halide salt" is a salt comprising an anion derived from a halogen.

In the context of the present invention, the term "multivalent" is intended to be understood as the property of a metal to form multivalent cations, i.e. cations having a valency of more than 1. For example, a multivalent cation may be divalent (i.e.: have a charge of +2), trivalent (i.e.: have a charge of +3), tetravalent (i.e.: have a charge of +4), or higher. Examples of multivalent cations include, but are not limited to, Mg²⁺, Ca²⁺, Al³⁺, and Fe³⁺.

In the context of the present invention, the term "multivalent halide salt" is intended to be understood as an ionic compound consisting of a multivalent cation and at least one halide anion. Examples include MgClz, CaClz, CaBr₂, and FeCl₃.

In the context of the present invention, the term "at least one multivalent halide salt" is intended to be understood as exactly one multivalent halide salt or a mixture of 2 or more multivalent halide salts, such as a mixture of MgClz and CaClz.

In the context of the present invention, the term "hydrophobic interaction chromatography" (HIC) is intended to be understood as a type of liquid chromatography used to separate and purify nucleic acids, polypeptides, and other biomolecules based on their hydrophobicity. A hydrophobic interaction chromatography generally comprises a mobile phase and a stationary phase. For example, the mobile phase may be a flowing liquid and the stationary phase may consist of a solid chromatography support onto which hydrophobic ligands are covalently attached. For example, the mobile phase may contain an aqueous solution with varying concentrations of salt.

In the context of the present invention, the term "mobile phase modifier" is intended to be understood as a chemical agent added to the mobile phase of a chromatography to influence the interaction between the stationary phase, the mobile phase, and the compound of interest. For example, the mobile phase modifier may reduce the hydrophobic interactions between the compound of interest and the stationary phase, facilitating the elution or flow-through of the compound from/through the chromatography support. Alternatively, the mobile phase modifier may enhance the hydrophobic interactions, thereby promoting the retention of the target molecule in the stationary phase while allowing other compounds to flow through the column.

The research leading up to the invention has shown that multivalent halide salts, which are traditionally classified as chaotropic salts, can be successfully used as a mobile phase modifier in hydrophobic interaction chromatography. This result is especially surprising since chaotropic salts are known to disrupt the structure of water and weaken the hydrophobic interactions within and between molecules, which is why multivalent halide salts have not been considered for use in hydrophobic interaction chromatography. In accordance with the present disclosure, it is generally preferred that the at least one multivalent halide salt is used as the primary and/or only mobile phase modifier.

Using multivalent halide salts as mobile phase modifiers in hydrophobic interaction chromatography has the advantage that waste streams can be discarded without the risk of environmental damage due to eutrophication. At the same time, a dynamic binding capacity in HIC applications that is comparable to the one obtained with ammonium sulfate can be achieved by using multivalent halide salts, and they may be used to selectively retain and elute certain types of biomolecules, i.e. to separate a compound of interest from unwanted impurities such as endotoxins, for example.

The object named above is further solved in accordance with the present invention by a method for purifying biomolecules using hydrophobic interaction chromatography, the method comprising:
- passing a sample comprising the biomolecules through the chromatography support, wherein the sample further comprises at least one multivalent halide salt at a first concentration, and
- passing a first solution through the chromatography support, wherein the first solution comprises the at least one multivalent halide salt at a second concentration, wherein the second concentration is lower than the first concentration.

In the context of the present invention, the term "biomolecule" is intended to be understood as an organic molecule that is produced by a living organism or a synthetic procedure. For example, a biomolecule may be a nucleic acid such as DNA or RNA, or a polypeptide such as a peptide or a protein.

In the context of the present invention, the term "sample" is intended to be understood as a mixture of biomolecules that is introduced into a chromatography support for separation and purification based on their hydrophobic properties. Generally, the sample further comprises a buffer containing salt, which in accordance with the present invention is a multivalent halide salt. For example, a sample may further comprise impurities and/or contaminants such as endotoxins or other biomolecules which need to be removed during the separation and purification process.

In the context of the present invention, the term "chromatography support" is intended to be understood as the solid matrix or material within the chromatography column that serves as the foundation for the stationary phase. The support is typically functionalized with hydrophobic ligands that interact with the sample's hydrophobic molecules, facilitating their separation based on differences in hydrophobicity. For example, the chromatography support may be present in the form of a monolith, a membrane, packed or loose granules, fibers, or a porous gel. The chromatography support is also referred to herein as "support".

In the context of the present invention, the first solution may be understood as a wash or elution solution. In general, the first solution is an aqueous solution comprising at least one multivalent halide salt at a second concentration or multiple second concentrations (e.g., in case of a salt gradient) which is/are lower than the first concentration.

Therein, it is appreciated that a skilled person is capable of routinely optimizing the first and second concentrations depending on the binding and eluting behavior of the biomolecule of interest and the binding and eluting behavior of other components of the sample such as impurities and contaminants.

For example, the first concentration may be selected to allow the biomolecule of interest to flow through the chromatography support while more hydrophobic impurities/contaminants are bound by the chromatography support (flow-through mode). In this case, the chromatography support may subsequently be flushed with a washing solution comprising the at least one multivalent halide salt at approx. the first concentration, preferably at the same concentration as present in the sample, to remove any remaining unbound biomolecule of interest from the support. Subsequently, any specifically and non-specifically bound molecules such as impurities or contaminants may be removed from the chromatography support by flushing the chromatography support with a washing solution free from multivalent halide salt and/or a sanitizing solution which may comprise sodium hydroxide, for example.

For example, the first concentration may be selected to allow the biomolecule of interest to bind to the chromatography support while less hydrophobic impurities/contaminants pass through the chromatography support without binding to it (bind-elute mode). In this case, the chromatography support may subsequently be flushed with a washing solution comprising the at least one multivalent halide salt at approx. the first concentration (same concentration as the sample) or a higher concentration to remove any unbound impurities from the support. Subsequently, the biomolecule of interest is eluted from the support by passing through the first solution which contains the multivalent halide salt at a second concentration which is lower than the first concentration, e.g. a constantly lower concentration or a decreasing salt gradient. Without wishing to be bound by theory, it is currently assumed that the biomolecule is precipitated onto the support when present in a solution comprising the high first concentration of multivalent halide salt and is solubilized - and thus eluted from the support - in a solution comprising the lower second concentration of multivalent halide salt. After eluting the biomolecule of interest, any specifically and non-specifically bound molecules such as impurities or contaminants may be removed from the chromatography support by flushing the support with a washing solution free from multivalent halide salt and/or a sanitizing solution which may comprise sodium hydroxide, for example.

For example, before passing the sample through the chromatography support, the support may be flushed with a preconditioning buffer comprising the at least one multivalent halide salt, preferably at approx. the first concentration.

The object named above is further solved in accordance with the present invention by a use of a chromatography support in a method according to the invention.

In this respect, any suitable chromatography support described herein may be used in the hydrophobic interaction chromatography method according to the invention.

The object named above is further solved in accordance with the present invention by a Kit for the purification of biomolecules comprising a chromatography support comprising hydrophobic ligands, and at least one multivalent halide salt.

In this respect, any chromatography support described herein that is suitable for hydrophobic interaction chromatography may be part of the kit according to the invention. Moreover, it is generally appreciated that the multivalent halide salt is present in the kit in a sufficient amount or concentration that allows for carrying out a method according to the invention using the kit, in particular a method where the at least one multivalent halide salt is used as mobile phase modulator in a hydrophobic interaction chromatography workflow.

Various embodiments of the use of multivalent halide salts in HIC, the HIC method, the use of a chromatography support in the HIC method and the Kit are described in the following. The individual embodiments are in each case individually applicable to the above-named aspects of the present invention. The individual embodiments may furthermore be combined with each other at will.

In an exemplary embodiment, the at least one multivalent halide salt comprises divalent and/or trivalent metal cations, preferably Mg, Ca, Sr, Al, Fe, and/or Ba, more preferably Mg and/or Ca cations.

For example, the multivalent halide salt may comprise metal cations from the elements in the second or 13^{th} group of the periodic table, as well as from transition metals (i.e.: group 3 to 12) from the 4^{th} period. Exemplary cations include Mn²⁺, Fe²⁺, Fe³⁺, Co²⁺, Co³⁺, Ni²⁺, Cu²⁺, Zn²⁺, Al³⁺, Ba²⁺, Sr²⁺, Be²⁺, Ca²⁺, and Mg²⁺.

It is generally appreciated that the multivalent halide salt needs to have a sufficient solubility in water to be used according to the invention.

In an exemplary embodiment, the at least one multivalent halide salt comprises F anions, Cl anions, Br anions, I anions, or At anions, or any combination thereof, preferably Cl anions.

For example, a suitable multivalent halide salt may be selected from the group consisting of CaClz, MgClz, MgBr₂, CaBr₂, CaI₂, MgIz, FeI₂, FeBrs, BaBr₂, and BaI₂.

In one embodiment, the at least one multivalent halide salt is MgClz and/or CaClz.

In an embodiment, the at least one multivalent halide salt is present at a total maximum concentration of 0.25 M - 5.0 M.

In the context of the present invention, the term "total (...) concentration" is intended to be understood as the sum of concentrations of all multivalent halide salts. For example, if 2 M MgClz and 1 M CaClz are present, the total concentration is 3 M multivalent halide salt.

In the context of the present invention, the term "maximum concentration" is intended to be understood as the highest concentration value in which the multivalent halide salt is used. For example, if the multivalent halide salt is used in a first solution at a concentration of 4 M and in a second solution at a concentration of 2 M, the maximum concentration is 4 M. For example, in a decreasing salt gradient starting at 4 M and ending at 0.8 M, the maximum concentration is 4 M.

For example, the total maximum concentration may correspond to the concentration of a saturated solution of the at least one multivalent halide salt in water.

In the research leading up to the invention, it was found that in the context of hydrophobic interaction chromatography, the dynamic binding capacity of biomolecules such as plasmid DNA (pDNA) is dependent on multivalent halide salt concentration in the mobile phase. For example, at neutral pH, a dynamic binding capacity allowing for selecting supercoiled pDNA over open circular pDNA may be achieved in a concentration range of 1.5 to 2.5 M MgClz or 1.25 to 2 M CaClz.

In an exemplary embodiment, the at least one multivalent halide salt is present in at least one buffer having a pH value in the range from 2.5 - 11.

In this context, the term "buffer" is meant to include any aqueous solution, which may further comprise a buffering compound and/or a biomolecule of interest (also referred to as a "sample").

In the research leading up to the invention, it was found that in the context of hydrophobic interaction chromatography, the dynamic binding capacity for biomolecules such as pDNA achieved by use of multivalent halide salts depends in part on the pH value. For example, at an acidic or alkaline pH value, the at least one multivalent halide salt may provide a high biomolecule binding capacity at a lower concentration than when used at neutral pH.

For example, the pH value may be selected depending on the biomolecule of interest, e.g. according to an isoelectric point of said biomolecule.

For example, if the at least one multivalent halide salt is present in more than one buffer, these pH values of these buffers may differ from one another or may be the same.

In an exemplary embodiment, the mobile phase comprises less than 0.5 mol/L kosmotropic salt, preferably less than 0.05 mol/L kosmotropic salt, more preferably wherein the mobile phase is essentially free from kosmotropic salt.

In the context of the present invention, the term "kosmotropic salt" is intended to be understood as a salt whose ions have a tendency to stabilize and order water molecules, promoting stronger water-water interactions, wherein the anion is generally understood to have the primary influence. The degree of kosmotropic influence is determined by the commonly known Hofmeister series. Kosmotropic salts are traditionally used in hydrophobic interaction chromatography. Exemplary kosmotropic salts include, but are not limited to, ammonium sulfate, sodium sulfate, potassium phosphate, sodium citrate, and potassium citrate. Therein, it is generally appreciated that the concentration of kosmotropic salt corresponds to the concentration of kosmotropic anions (sulfate, phosphate, citrate, pyrophosphate) in the mobile phase.

In this context, the term "essentially free" is intended to be understood in that the mobile phase containing multivalent halide salt contains only trace amounts of kosmotropic salt or none at all, to the extent that the remaining kosmotropic salt has no significant impact on the chemical, physical, or functional properties of the mobile phase. For example, "essentially free" may mean that the mobile phase contains kosmotropic salt in a concentration of 10 mM or less, 5 mM or less, or 1 mM or less.

Surprisingly, the research leading up to the invention has shown that mixtures of kosmotropic salts and the multivalent halide salts of the present invention are not preferred in concentrations sufficient for hydrophobic interaction chromatography, because they often result in precipitation of insoluble species. For example, a mixture of calcium chloride (CaCl₂) and the kosmotropic salt ammonium sulfate leads to the precipitation of calcium sulfate even at starting concentrations as low as 0.1 M which are insufficient for HIC applications.

In an exemplary embodiment, the invention may be used for the purification of biomolecules, wherein preferably the biomolecules are selected from the group consisting of nucleic acids, for example plasmid DNA, polypeptides, viruses, and virus-like particles.

In the context of the present invention, the term "nucleic acid" is intended to be understood as polynucleotides consisting of ribonucleic acids (RNA) or deoxyribonucleic acids (DNA). For example, nucleic acids may be of natural or synthetic origin. For example, nucleic acids may encompass different topological forms such as supercoiled, open circular or linear constructs. A particular example of a nucleic acid is a plasmid, which is a circular polynucleotide that is usually present in supercoiled form.

In the context of the present invention, the term "polypeptide" is intended to mean linear or branched chains of amino acids linked together by peptide bonds. For example, a polypeptide may have a specific three-dimensional structure or may be unfolded, i.e. have a non-specific structure. A particular example of a polypeptide is a protein, i.e., a polypeptide which performs a biological function.

In the context of the present invention, the term "virus" is intended to mean a submicroscopic infectious agent composed primarily of genetic material (either DNA or RNA) encased within a protein coat (capsid). Some viruses further comprise an additional lipid envelope.

In the context of the present invention, the term "virus-like particle" (VLP) is intended to refer to a molecular structure that closely resembles a virus but lacks the viral genome, making it non-infectious. For example, a virus-like particle may be composed of viral proteins that can self-assemble into structures that mimic a virus's capsid or envelope but do not contain the nucleic acid required for replication.

In an exemplary embodiment of the chromatography method according to the invention, the biomolecule is a nucleic acid, preferably plasmid DNA, and/or a polypeptide.

In an exemplary embodiment of the chromatography method according to the invention, the sample additionally comprises a buffer to maintain the pH in the range of 2.5 - 11.

Therein, the compound and concentration of the buffer compound is not crucial. It is, however, preferred that the buffering compound does not include a kosmotropic anion such as sulfate or phosphate as these tend to precipitate with the multivalent halide salts according to the invention.

In an exemplary embodiment of the method according to the invention, the at least one multivalent halide salt is present in the sample at a total concentration of 0.25 - 5.0 M.

In an exemplary embodiment of the method according to the invention, also referred to herein as "bind-elute mode", the at least one multivalent halide salt is present in the sample at a total concentration of 0.25 - 5 M. For example, when passing the sample through a chromatography support, the biomolecule of interest is retained on the support while impurities and contaminants end up in the flowthrough or bind to the chromatographic support.

In this embodiment, it is preferred that passing the first solution through the chromatography support comprises applying a decreasing salt gradient of the at least one halide salt, whereby impurities and contaminants are gradually removed from the chromatography support while the biomolecule of interest stays bound to the support. Once the salt concentration reaches a certain value which depends on the biomolecule of interest, it separates from the support and can be collected in the flowthrough. By further decreasing the salt concentration, the remaining impurities and contaminants are at least partially removed from the support.

In this embodiment, it is preferred that the concentration of the multivalent halide salt is decreased from an initial concentration of 0.25 - 5 M to a final concentration of 0 - 1 M. Therein preferably, the decrease is carried out in a continuous (e.g.: linear, exponential or sigmoidal decrease of the salt concentration) or step-wise (e.g.: steps of 0.2 M salt concentration) fashion.

In an exemplary embodiment of the method according to the invention, the chromatography support is suitable for hydrophobic interaction chromatography or reverse-phase chromatography. Therein, it is preferred that the chromatography support comprises neutral ligands selected from the group consisting of alkyl, aryl, cycloalkyl, hydroxy, epoxy, ether, amide and ester, and/or multimodal hydrophobic-hydrogen bonding ligands such as pyridine. It is further preferred that the chromatography support is a porous particle support, a membrane support, a monolith support or a nanofiber support.

For example, the chromatography support may be a hydrophobic resin, e.g. comprising porous particles. As described herein, a hydrophobic resin is any material to which the target biomolecule (e.g., an oligonucleotide) will bind such that it can be separated from impurities and contaminants in a method according to the invention. For example, the hydrophobic adsorbent may include hydrophilic carbohydrates, such as cross-linked agarose and synthetic copolymer materials. According to the present invention, the hydrophobic adsorbent comprises either phenyl or hexyl. For example, Hexyl650C is a suitable hydrophobic adsorbent. For example, a hydrophobic adsorbent may comprise butyl. Furthermore, the hydrophobic adsorbent may be packed into a column or similar structure at any bed height.

In an exemplary embodiment, the chromatography support comprises hydrophobic ligands selected from the group consisting of alkyl, aryl and cycloalkyl.

In the context of the present invention, the term "alkyl" is intended to refer to a fully saturated branched or unbranched hydrocarbon moiety. For example, the alkyl may comprise 1 to 20 carbon atoms, more preferably 1 to 16 carbon atoms, 1 to 10 carbon atoms, 1 to 6 carbon atoms, or 1 to 4 carbon atoms. In some embodiments, an alkyl comprises from 6 to 20 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, or n-decyl.

In the context of the present invention, the term "alkenyl" is intended to refer to an unsaturated hydrocarbon group which may be linear or branched and has at least one carbon-carbon double bond. Alkenyl groups with 2-6 carbon atoms may be preferred. The alkenyl group may contain 1, 2 or 3 carbon-carbon double bonds, or more. Examples of alkenyl groups include ethenyl, n-propenyl, isopropenyl, n-but-2-enyl, n-hex-3-enyl and the like.

In the context of the present invention, the term "alkynyl" is intended to refer to an unsaturated hydrocarbon group which may be linear or branched and has at least one carbon-carbon triple bond. Alkynyl groups with 2-6 carbon atoms may be preferred. The alkynyl group may contain 1, 2 or 3 carbon-carbon triple bonds, or more. Examples of alkynyl groups include ethynyl, n-propynyl, n-but-2-ynyl, n-hex-3-ynyl and the like.

In the context of the present invention, the term "aryl" is intended to refer to monocyclic, bicyclic or tricyclic aromatic hydrocarbon groups having from 6 to 14 carbon atoms in the ring portion. In one embodiment, the term aryl refers to monocyclic and bicyclic aromatic hydrocarbon groups having from 6 to 10 carbon atoms. Representative examples of aryl groups include phenyl, naphthyl, fluorenyl, and anthracenyl. The term "aryl" is also intended to refer to a bicyclic or tricyclic group in which at least one ring is aromatic and is fused to one or two non-aromatic hydrocarbon ring(s). Nonlimiting examples include tetrahydronaphthalene, dihydronaphthalenyl and indanyl. An "arylalkyl" is intended to refer to an aryl group linked via an alkylene linker to the reminder of the molecule. An "alkaryl" is intended to refer to an alkyl group linked via an arylene linker to the reminder of the molecule.

In the context of the present invention, the term "cycloalkyl" is intended to refer to a fully saturated cyclic hydrocarbon moiety. For example, the cycloalkyl may comprise 3 to 12 carbon atoms, more preferably 3 to 8 carbon atoms, or 3 to 6 carbon atoms. Representative examples of cycloalkyl include, but are not limited to, cyclopropyl, cyclobutyl, cyclopentyl, cyclohexyl, cycloheptyl, and cyclooctyl. Cycloalkyl groups may be substituted or unsubstituted, and the ring may be either monocyclic or polycyclic.

In an exemplary embodiment of the Kit according to the present disclosure, the at least one multivalent halide salt is present in a buffer solution, preferably in the form of a stock solution.

For example, the buffer solution, or preferably the stock solution, may comprise the at least one multivalent halide salt at a concentration sufficient for carrying out a hydrophobic interaction chromatography when mixed with the sample to be used.

In an exemplary embodiment of the Kit according to the present disclosure, the hydrophobic ligands are selected from the group consisting of alkyl, aryl and cycloalkyl or any combinations thereof.

Further features and advantages of the aspects and embodiments of the invention emerge from the following description of experimental data with reference to the attached figures.

In the figures:
Fig. 1 shows a preparative chromatogram for binding of pDNA (4.7 kbp) containing a mixture of supercoiled (SC) and open circular (OC) isoforms, onto CIM^{®} C4 HLD monolith chromatographic column in presence of 1.75 M CaClz at pH 7.2 used as a mobile phase modifier.
Fig. 2 shows an analytical HPLC chromatogram using the in-process samples from purification shown in Fig. 1.
Fig. 3 shows an agarose gel comprising the chromatography fractions shown in Fig. 2.
Fig. 4 shows the binding capacity for various concentrations of CaClz, ammonium sulfate and MgClz used as mobile phase modifiers enabling binding of 4.7 kbp pDNA to a CIM^{®} C4 HLD monolithic chromatographic column.
Fig. 5 shows the binding capacity for binding of pDNA (4.7 kbp) onto a CIM^{®} C4 HLD monolithic chromatographic column, while 1.5 M ammonium sulfate or 1.5 M CaClz are present as mobile phase modifiers at different pH values.
Fig. 6 shows a preparative chromatogram for binding of pDNA (4.7 kbp) containing a mixture of supercoiled (SC) and open circular (OC) isoforms to CIM^{®} C4 HLD monolith chromatographic column in the presence of 2.25 M MgClz at pH 7.2 used as a mobile phase modifier.
Fig. 7 shows an analytical HPLC chromatogram using the in-process samples from the purification shown in Fig. 6.
Fig. 8 shows a preparative chromatogram for the protein bovine serum albumin (BSA) binding to CIMmultus^{®} C4 A monolith in the presence of 2.25 M CaClz at pH 7.2 as mobile phase modifier, and elution in 0 M CaClz.
Fig. 9 shows a preparative chromatogram for binding of 4.7 kbp pDNA containing a mixture of SC and OC isoforms to CIMmultus^{®} C4 HLD 40 mL (2 µm channel diameter) monolith in the presence of 2 M CaClz at pH 7.2 as a mobile phase modifier.

Exemplary, non-limiting working examples of the invention are disclosed in the following. These examples are intended to illustrate that the invention leads to beneficial effects over a broad range of conditions and in combination with a large variety of chromatography paradigms, biomolecules, and chromatography supports.

### Materials and Preparations

Buffer solutions were freshly prepared with double-distilled water (ddHzO) and analytical grade reagents. They were filtered through a 0.2 µm PES filter (Thermo Fisher Scientific, Nalgene Rapid-Flow). Trizma-base (TRIS), MES hydrate, CaClz x 2H₂O, MgClz x 6H₂O, hydrochloric acid (HCl), (NH₄)₂SO₄ and agarose were from Sigma-Aldrich, NaCl and NaOH were from Honeywell, disodium salt of ethylenediaminetetraacetic acid (EDTA) was from Kemika and glycine was from Merck. The ddHzO used for all experiments was prepared with Adrona. Plasmid-containing E. coli pastes were prepared as described previously (Kos et al., 2021) and purified by clarification and DEAE chromatography as described elsewhere (Cernigoj & Strancar, 2021; Smrekar et al., 2010). Pre-purified plasmid pUCBS4.7 (4.7 kbp, 25% OC, 75% SC) was stored in 10 mM TRIS, 1 mM EDTA, pH 8.0 or in 50 mM TRIS, 10 mM EDTA, 0.75 M NaCl, pH 7.2 (used for scale-up run - see example 6 below).

Chromatographic separations were performed on PATfix^{™} (Sartorius BIA Separations), equipped with UV detection (260 and 280 nm wavelength, 10 mm optical path length), conductivity and pH probes, or were performed on Äkta Pure 150 (Cytiva), equipped with UV detection (260 and 280 nm, 2 mm optical path length), conductivity and pH probes. PATfix (Sartorius BIA Separations) or UNICORN (Cytiva) software were used for instrument control, data acquisition and data analysis. Flowthrough fractions were analyzed by UV-Vis spectrophotometry at wavelengths of 260 and 280 nm (NanoDrop One, Thermo Fischer Scientific) to determine the concentration of pDNA. Isoform purity for each of the fractions was determined by CIMac^{®} pDNA AEX HPLC analysis (PATfix, Sartorius BIA Separations) as described previously (Pavlin et al., 2023). Mupid^{®}-One (Advance Co., Ltd.) was used for gel electrophoresis. QuantStudio^{™} 5 (Thermo Fisher Scientific) was used for qPCR. Endosafe^{®} nexgen-PTS^{™} (Charles River Laboratories) was used for endotoxin testing according to the manufacturer's instructions.

Wash solution (free from multivalent halide salt) was 50 mM Tris, pH 7.2. Loading solutions contained 50 mM Tris, pH 7.2 with different (first) concentrations of CaClz, MgClz or ammonium sulfate (AS) equal to the concentration present in the sample to be analyzed. In experiments with AS, buffers further comprised 10 mM EDTA, which acts as a chelator for divalent metal ions and was therefore avoided when CaClz or MgClz was used. In experiments at pH 5.5, 50 mM MES was used as a buffering compound instead of Tris. At pH 3.5 and pH 10, 50 mM glycine was used as a buffering compound instead of Tris. At pH 9, 50 mM Tris was used as buffering compound.

Before each run, columns were sanitized, regenerated and equilibrated with 10 column volumes (CV) of cleaning-in-place sanitizing solution (1 M NaOH, contact time 15 min), followed by 10 CV of ddHzO, ≥3 CV of wash solution and ≥3 CV of loading solution.

All chromatographic experiments were performed at least in triplicates.

### Example 1: Dynamic binding capacity of plasmid DNA when using multivalent halide salts as mobile phase modifier

The example was carried out to determine the binding capacity and the selectivity of the chromatographic support when multivalent halide salt CaClz at neutral/physiological pH values (exemplary pH: 7.2) is used as a mobile phase modifier in hydrophobic interaction chromatography. As a chromatography support, a hydrophobic monolithic column (CIMultus^{®} C4 HLD 1 mL, 2 µm channel diameter, Sartorius BIA Separations) comprising high density butyl ligands was used. To a sample comprising 0.2 mg/mL of plasmid DNA, of which 75 % was present in supercoiled form (sc pDNA; biomolecule of interest) and 25 % was present in open circular form (oc pDNA; impurity), CaClz was added to the sample from a stock solution to achieve multiple total concentrations in the range of 1.25 to 2.5 M. Additionally, samples comprising 1.25 to 2.5 M ammonium sulfate at pH 7.2 were generated.

The samples comprising CaClz or ammonium sulfate, respectively, were passed through the chromatography support at a flow rate of 2.5 column volumes per minute (CV/min). After a respective sample had been loaded onto the chromatography support, the support was flushed with a solution containing the same total concentration of CaClz or ammonium sulfate at 2.5 CV/min, and thereafter a first solution comprising 0.9 M CaClz or ammonium sulfate was passed through the support at 1 CV/min, and thereafter the support was flushed with a wash solution ("STRIP") free from multivalent halide salt (0 M CaClz) at 1 CV/min. In each case, the flowthrough fractions were collected for further analysis.

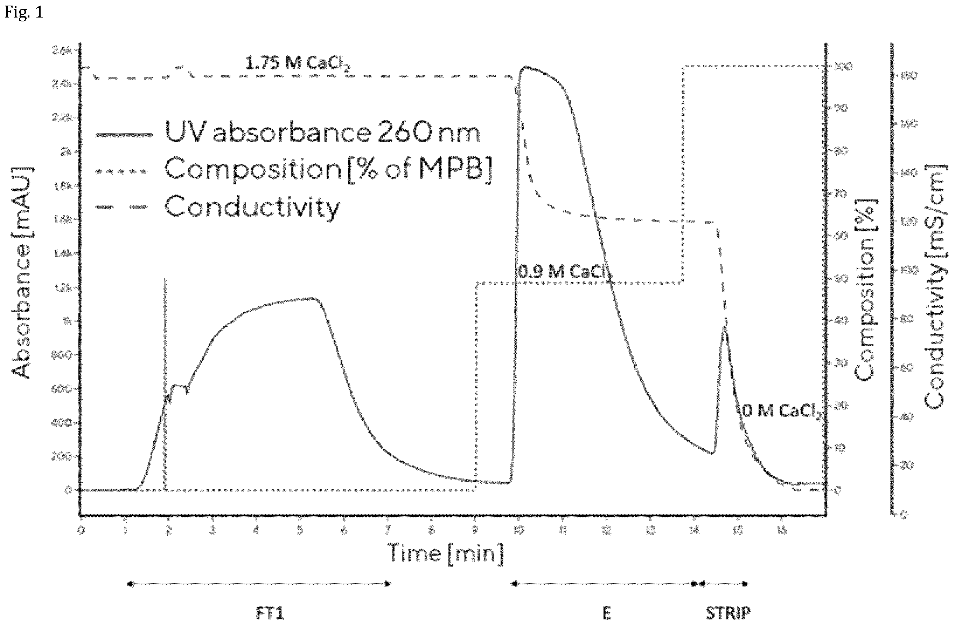

Parameters such as the flow rate of liquid passing through the chromatography support, as well as the salt concentration and conductivity were also tracked. Fig. 1 shows an exemplary chromatogram depicting the UV absorbance value (continuous line), the salt concentration (dotted line) and the conductivity (dashed line) over the course of the chromatography run, where 1.75 M CaClz at pH 7.2 was used as a mobile phase modifier enabling binding of pDNA having a length of 4.7 kilobasepairs (kbp) to the CIM^{®} C4 HLD monolith. As documented in Fig. 1, the sample was loaded onto the support between the 1-minute and 5-minute timestamps, the support was washed approx. between the 5-minute and 9-minute timestamps, the first solution was passed through the column approx. between the 9-minute and 14-minute timestamps, and the wash solution was applied approx. between the 14-minute and 17-minute timestamps.

Fig. 1 documents that a breakthrough of biomolecules occurred, which is indicated by an increase of absorbance starting between the 1-minute and 2-minute timestamps. Plasmid DNA was eluted from the support by applying the first solution, which is indicated by the large increase in the absorbance between approx. the 10-minute and 12-minute timestamps. More pDNA was removed from the support by applying the wash solution, which is indicated by a smaller absorbance peak around the 15-minute timestamp. This indicates that under binding conditions, the SC isoform was retained on the column while the OC isoform did not bind and was recovered in flow-through ("FT1"), and that SC was eluted upon reducing the concentration of CaClz to 0.9 M ("E").

The binding capacity for pDNA was determined as the amount of biomolecule that can be loaded onto the chromatography support in certain conditions. The binding capacity may be interpreted as the amount of biomolecule that can bind to a chromatography support until a breakthrough is observed. For example, the binding capacity can be determined by calculating the amount of biomolecule that is eluted from the chromatography support. Through the present disclosure, the binding capacity is expressed as the amount (in mg) of biomolecule that is retained by 1 mL of chromatography support, the unit is mg/mL.

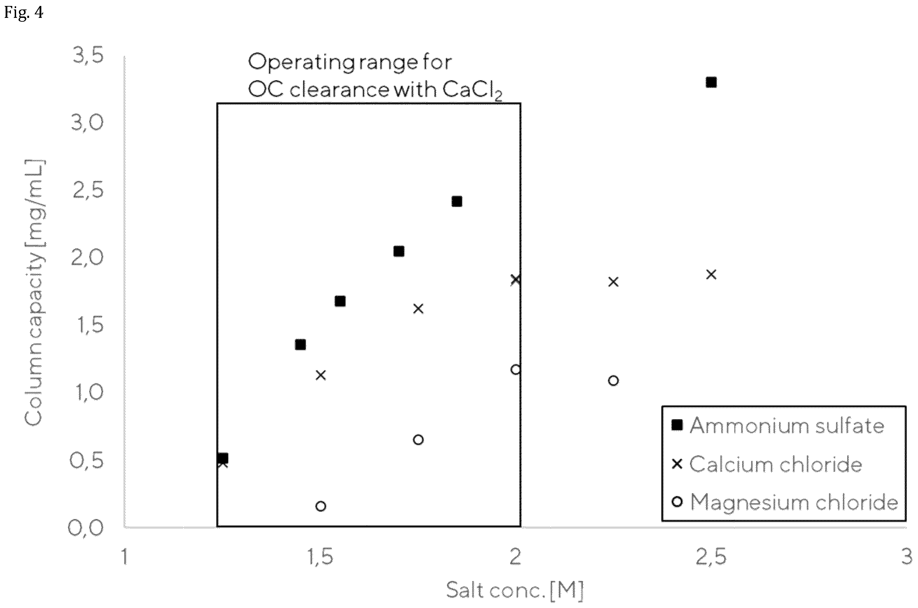

Fig. 4 shows the binding capacity obtained when using different concentrations of CaClz (crosses), MgClz (circles) and ammonium sulfate (squares). For CaClz, Fig. 4 shows that the binding capacity for pDNA at neutral pH 7.2 increases between 1.25 and 2 M while remaining constant above 2 M. For ammonium sulfate, the binding capacity for pDNA at pH 7.2 increases up until a concentration of 2.5 M, which is a concentration typically used in hydrophobic interaction chromatography for pDNA. Moreover, the binding capacity of C4 HLD monolith for pDNA in a CaClz solution is approx. 25 % lower than in an ammonium sulfate solution. The box shown in Fig. 4 indicates the operating range of salt concentrations in the mobile phase at which a successful separation of sc pDNA from oc pDNA can be achieved (also termed "OC clearance") and the binding capacity increases approx. linearly with the concentration of multivalent halide salt.

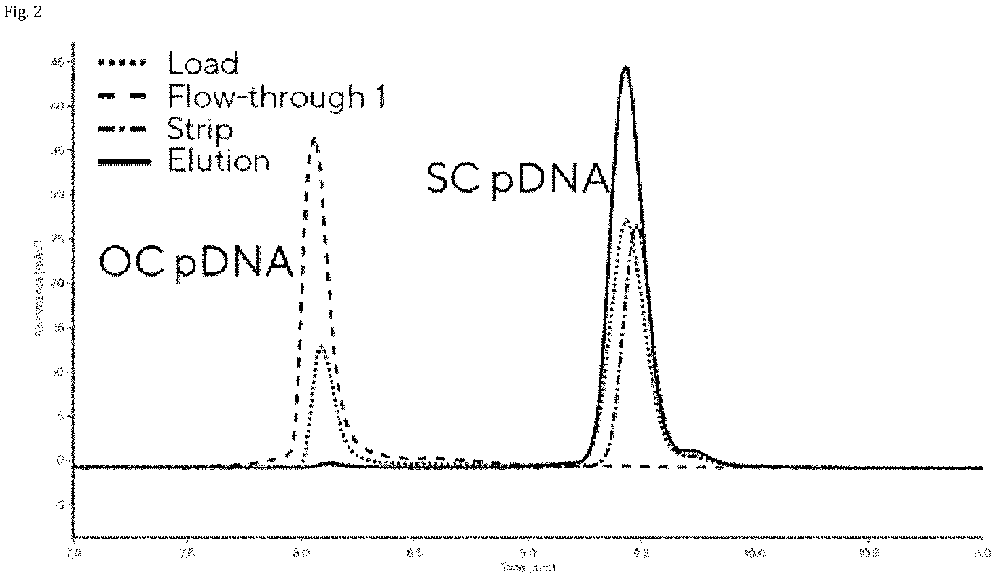

Fig. 2 shows HPLC chromatograms for four process samples: the loaded sample containing the biomolecule of interest and impurities ("Load"; dotted line), the flowthrough after passing the sample through the support ("flow-through 1" or "FT1"; dashed line), the flowthrough after passing the first solution through the support ("Elution" or "E"; continuous line), and the flowthrough after passing the wash solution through the support ("Strip"; dotted-dashed line). 1.75 M CaClz at pH 7.2 was used as a mobile phase modifier enabling binding of 4.7 kbp pDNA to the CIM^{®} C4 HLD monolith.

The HPLC analysis revealed two distinct peaks. In a previous experiment not shown here, the right peak according to Fig. 2 was attributed to the biomolecule of interest, sc pDNA, and the left peak was attributed to the impurity, oc pDNA. Fig. 2 shows that the load fraction contained both sc pDNA and oc pDNA, whereas the FT1 fraction contained >99 % oc pDNA (impurity), and the E fraction contained >95 % sc pDNA (biomolecule of interest). Thereby, the HPLC analysis indicates that biomolecules of interest can be successfully separated from impurities and/or contaminants by the method according to the invention.

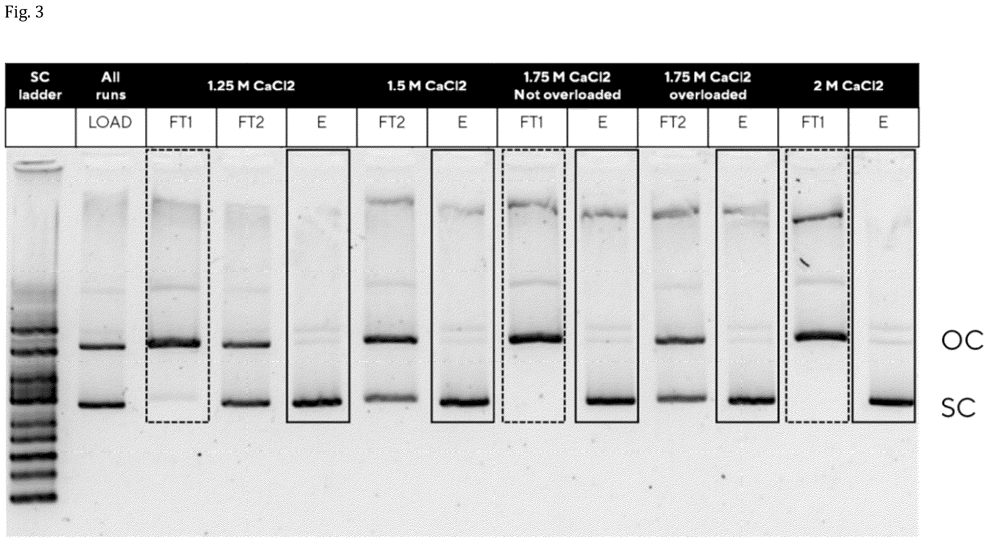

This result was confirmed by agarose gel electrophoresis using a gel comprising 1 % agarose and SYBRgold in 1x Tris-Acetate-EDTA (TAE) buffer. The Load, flow-through 1 (FT1) and/or flow-through 2 (FT2 - corresponding to first break-through of SC isoform through the column) and elution (E) fractions from purification when OC/SC pDNA containing samples further containing a total concentration of 1.25 to 2 M CaClz at pH 7.2 were subjected to standard gel electrophoresis (100 V, 60 min) together with a DNA ladder (SC ladder, NEB). The results are shown in Fig. 3. In a previous experiment not shown here, the lower band according to Fig. 3 had been attributed to the biomolecule of interest (sc pDNA, "SC"), and the higher band to the impurity/contaminant (oc pDNA, "OC").

Fig. 3 shows that the oc pDNA predominantly did not bind to the support at all, which is indicated by the respective bands in the FT1 (dashed-frame boxes) and FT2 samples. Moreover, the sc pDNA was completely bound by the chromatography support, which is indicated by the fact that a sc pDNA band was only visible in the FT2 fractions, i.e. when the support was overloaded with sample, i.e. when the binding capacity of the support was not sufficient to bind the entirety of sc pDNA biomolecules contained in the sample. Fig. 3 further confirms that in the range between 1.25 and 2 M CaClz, independently from the specific CaClz concentration in the sample, predominantly sc pDNA and almost no oc pDNA was eluted by the first solution, which is indicated by the strong sc pDNA band and very weak oc pDNA band in the E samples (continuous-frame boxes).

The experiment shows that the multivalent halide salt CaClz can be successfully used for separating a biomolecule of interest from an impurity in hydrophobic interaction chromatography, e.g. when using a CIM^{®} HLD monolith. At pH 7.2, CaClz provides selective binding conditions for sc pDNA at least in the concentration range between 1.25 M and 2 M, and the binding capacity for pDNA is comparable to the one obtained with known methods based on ammonium sulfate as the mobile phase modifier. In a solution comprising CaClz and a mixture of sc pDNA and oc pDNA, the hydrophobic chromatography support retained predominantly sc pDNA, and the sc pDNA could be further isolated from oc pDNA impurities by elution with a first solution containing a lower second concentration of the multivalent halide salt, in this case 0.9 M CaClz. Thereby, the present invention provides a workflow for hydrophobic interaction chromatography which is based on environmentally friendly and easy-to-handle multivalent halide salts.

### Example 2: Hydrophobic interaction chromatography using magnesium chloride as mobile phase modifier

Preparative runs (loading and wash steps) and analytics for experiments in this example were performed the same way as experiments in Example 1, except that CaClz was replaced with MgClz as a mobile phase modifier.

The example was carried out to determine the binding capacity and the selectivity of the chromatographic support when multivalent halide salt MgClz at neutral pH 7.2 is used as a mobile phase modifier in hydrophobic interaction chromatography. As a chromatography support, a hydrophobic monolithic column (CIMultus^{®} C4 HLD 1 mL, 2 µm channel diameter, Sartorius BIA Separations) comprising high density butyl ligands was used. To a sample comprising 0.2 mg/mL of plasmid DNA, of which 75 % was present in supercoiled (sc pDNA) form (biomolecule of interest) and 25 % was present in open circular (oc pDNA) form (impurity), MgClz was added as a stock solution to achieve multiple total concentrations in the range of 1.5 to 2.25 M.

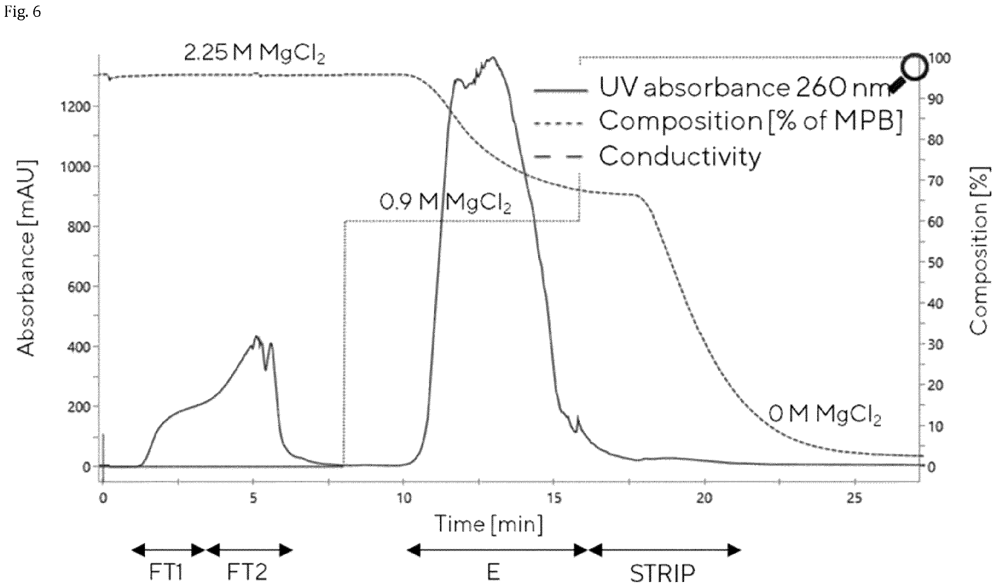

Fig. 6 shows an exemplary chromatogram depicting the UV absorbance value (continuous line), the salt concentration (dotted line) and conductivity (dashed line) over the course of the chromatography run, where 2.25 M MgClz at pH 7.2 was used as a mobile phase modifier enabling binding of 4.7 kbp pDNA to the CIM^{®} C4 HLD monolith. Under binding conditions, the SC isoform was retained on the column while OC isoform did not bind and was recovered in flow-through (FT1/FT2). SC pDNA was eluted upon reducing the concentration of MgClz to 0.9 M (first solution: 0.9 M MgClz, 50 mM Tris, pH 7.2). According to Fig. 6, the sample was loaded onto the support between the 1-minute and 5-minute timestamps, the support was washed approx. between the 5-minute and 7.5-minute timestamps, the first solution was passed through the column approx. between the 7.5-minute and 16-minute timestamps, and the wash solution was applied approx. between the 16-minute and 27-minute timestamps.

Fig. 6 shows that two breakthroughs of biomolecules occurred, which is indicated by increases of absorbance starting between the 1-minute and 2-minute timestamps and at the 4-minute timestamp. Without wishing to be bound by theory, it is currently assumed that the first breakthrough is of oc pDNA, which is bound to a lesser degree in this setup, and the second breakthrough is of sc pDNA due to the column capacity being exceeded. Plasmid DNA was eluted from the support by applying the first solution, which is indicated by the large increase in the absorbance between approx. the 10-minute and 15.5-minute timestamps. More pDNA was removed from the support by applying the wash solution, which is indicated by a broader feature around the 18-minute timestamp.

Fig. 4 shows binding capacity values obtained by using different concentrations of MgClz (circles) besides results for CaCl₂ (crosses), and ammonium sulfate (squares). For MgClz, Fig. 4 shows that the binding capacity for pDNA at pH 7.2 increases between 1.5 and 2 M while remaining constant above 2 M. Moreover, the binding capacity of CIM^{®} C4 HLD monolith for pDNA in a MgClz solution is approx. 50 % lower than in an ammonium sulfate solution.

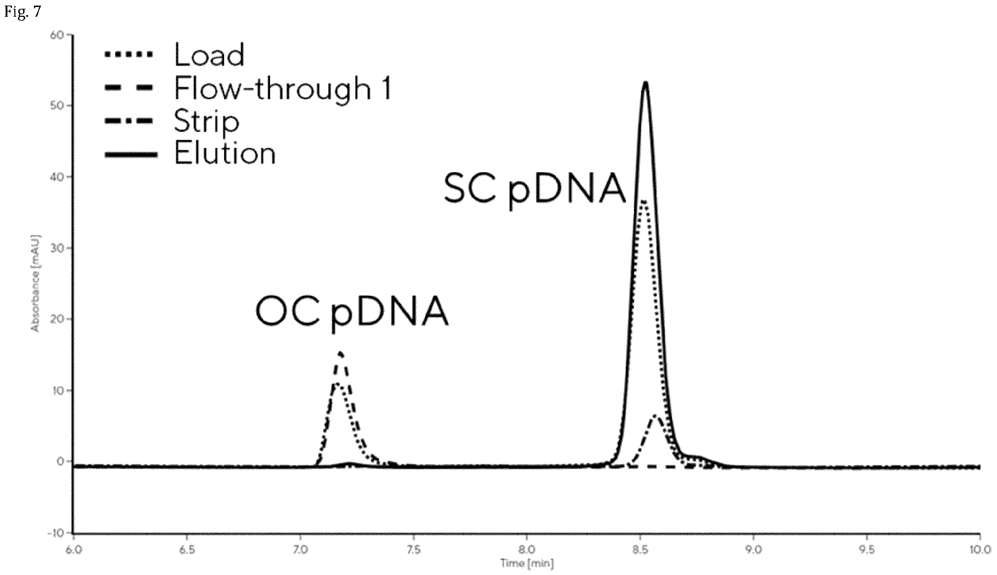

Fig. 7 shows an analytical HPLC chromatogram of the following in-process samples: load (dotted line), the flowthrough after passing the sample through the support ("flow-through 1" or "FT1"; dashed line), the flowthrough after passing the first solution through the support ("Elution" or "E"; continuous line), and the flowthrough after passing the wash solution through the support ("Strip"; dotted-dashed line). In the chromatography preceding the HPLC analysis shown in Fig. 7, 2.25 M MgClz at pH 7.2 was used as a mobile phase modifier enabling binding of 4.7 kbp pDNA to the CIM^{®} C4 HLD monolith.

The HPLC analysis revealed two distinct peaks. In a previous experiment not shown here, the right peak according to Fig. 7 had been attributed to the biomolecule of interest, sc pDNA, and the left peak was attributed to the impurity, oc pDNA. Fig. 7 shows that the load fraction contained both sc pDNA and oc pDNA, whereas the FT1 fraction contained >99 % oc pDNA and E fraction contained >95 % sc pDNA.

The experiment shows that the multivalent halide salt MgClz can be successfully used for separating a biomolecule of interest from an impurity in hydrophobic interaction chromatography on CIM^{®} HLD monolith. At pH 7.2, MgClz provides selective binding conditions for sc pDNA in a concentration window between 1.5 M and 2.25 M, and the binding capacity for pDNA is approximately 50 % lower than the one with ammonium sulfate. In a solution comprising MgClz and a mixture of sc pDNA and oc pDNA, the hydrophobic chromatography support retained predominantly sc pDNA, and the sc pDNA could be further isolated from oc pDNA impurities by elution with a first solution containing a lower concentration of 0.9 M MgClz.

### Example 3: Dynamic binding capacity at different pH values

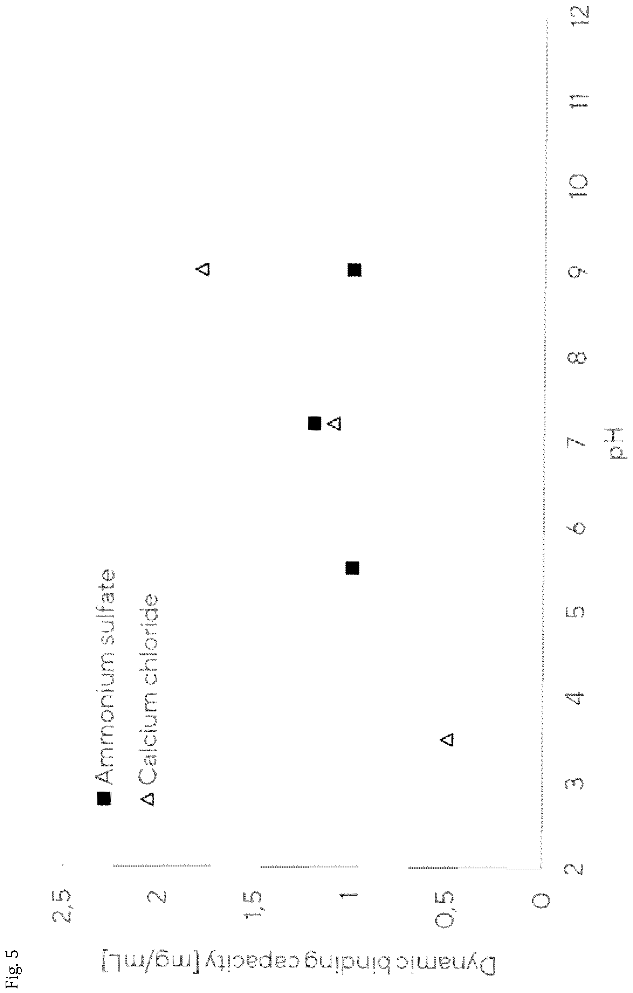

The aim of this example was to determine the binding capacity of biomolecules in hydrophobic interaction chromatography with multivalent halide salts as mobile phase modifiers at different pH values. Preparative runs (loading and wash steps) and analytics for experiments in this example were performed the same way as experiments in Example 1, except for different total concentrations of CaClz and different pH of solutions. An additional set of experiments was performed to check if a similar trend for pH dependency of binding capacity may be observed also with the known mobile phase modifier ammonium sulfate. Fig. 5 shows results for binding capacity from experiments where 1.5 M ammonium sulfate or 1.5 M CaClz at different pH values were used as loading conditions for 4.7 kbp pDNA to CIM^{®} C4 HLD monolith. The binding capacity was observed to be higher at more alkaline than at neutral pH using same concentration of CaClz. Thus, by increasing the pH value, a desired binding capacity may be achieved at a lower concentration of CaClz. This was confirmed in an experiment at pH 10 (not shown in Fig. 5, where only 0.5 M CaClz was used), and the resulting binding capacity (1.2 mg/mL) was comparable to the one achieved with 1.5 M CaClz at pH 7.2. On the other hand, the binding capacity was reduced at a pH value of 3.5 in comparison to neutral pH using same concentration of CaClz.

With respect to the binding capacity as a function of the pH value, a different trend than in case of CaClz was observed with ammonium sulfate: both at pH 5.5 and pH 9, the binding capacity was approximately 20 % lower (1.0 mg/mL) than at neutral pH (1.2 mg/mL), indicating that the maximum binding capacity value can be achieved at neutral pH and that the pH dependence of the binding capacity in ammonium sulfate does not follow the same pattern as CaClz. This strengthens the hypothesis that the mechanism enabling binding of pDNA in case of CaClz (chaotrope) may be significantly different from the mechanism in case of ammonium sulfate (kosmotrope). Also, the experiment shows that when using a method according to the invention, HIC can be performed over a larger range of pH values than with known mobile phase modifiers such as ammonium sulfate.

### Example 4: Removal of endotoxins by methods of the invention

Selected in-process samples from Examples 1 and 3 were analyzed for content of other important contaminants: wt.-% of genomic DNA (gDNA) and concentration of endotoxins (ETX) was tested as described elsewhere herein. Results in Table 1 show that a biomolecule of interest (here: pDNA) can be efficiently separated from impurities and contaminants (here: gDNA and ETX) using CaClz instead of the well-established ammonium sulfate. In all samples, the percentage of gDNA was reduced by at least a factor of 5.5, and the endotoxin content was reduced by more than 99.5 %.

The following table shows results for content of gDNA and endotoxins in the load and elution fractions from a few representative experiments from Examples 1 and 3.

| Loading conditions | ETX in load [EU/mL] | ETX in eluate [EU/mL] | gDNA in load [w/w] | gDNA in eluate [w/w] |
|---|---|---|---|---|
| 2 M CaCl₂, pH 7.2 | 2156 | <10 | 1.7 % | 0.3 % |
| 2 M CaCl₂, pH 8 | n.d. | n.d. | 2 % | 0.03 % |
| 1.5 M CaCl₂, pH 8.5 | n.d. | n.d. | 2 % | 0.15 % |
| 1.5 M CaCl₂, pH 9 | n.d. | n.d. | 2 % | 0.19 % |

| | | | | |
|---|---|---|---|---|
| n.d.: not determined | | | | |

### Example 5: Purification of proteins by methods of the invention

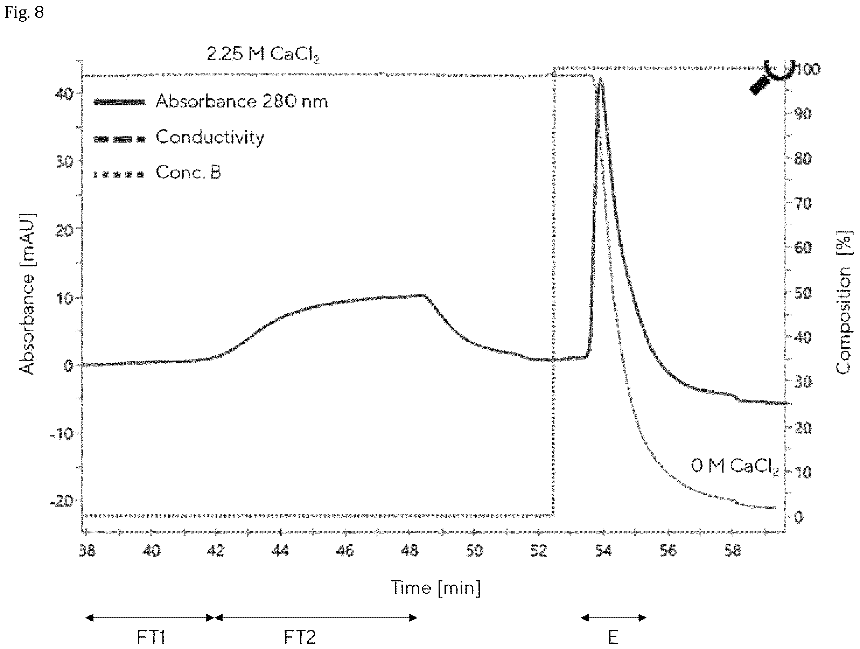

In this experiment, divalent chloride salts (CaClz) were used as mobile phase modifiers in hydrophobic interaction chromatography for protein purification. Bovine serum albumin (BSA) was used as a model protein. As proteins are known to be very hydrophobic in comparison to nucleic acids, a chromatographic support with lower hydrophobicity (CIMmultus^{®} C4 A, 2 µm channel diameter, Sartorius BIA Separations) was used to prevent potential irreversible binding of proteins, which is a known issue in hydrophobic interaction chromatography. A BSA sample comprising 2.25 M CaClz at pH 7.2 was prepared and loaded onto a chromatographic support until a breakthrough, representing unbound BSA, was observed. Fig. 8 shows a representative preparative chromatogram for the following chromatographic run (similar to the one described in Example 1 above): the sample was loaded onto a CIMmultus^{®} C4 A monolith chromatographic support between the 37-minute and 48-minute timestamps, the chromatographic support was washed between the 48-minute and 52.5-minute timestamps (wash solution: 2.25 M CaClz, 50 mM Tris, pH 7.2), and the first (elution) solution was passed through the column between the 52.5-minute and 60-minute timestamps (first solution: 0 M CaClz, 50 mM Tris, pH 7.2). Plotted are the UV absorbance value (continuous line), the salt concentration (dotted line) and the conductivity (dashed line).

Fig. 8 shows an increase of absorbance starting between the 42-minute and 43-minute timestamps, indicating that a breakthrough of BSA occurred (meaning that the binding capacity of the chromatographic support was exceeded). BSA was fully eluted from the support by applying the first solution, which is indicated by the increase in the absorbance between approx. the 53.5-minute and 56-minute timestamps. This example shows that CaClz can be used also as a mobile phase modifier in hydrophobic interaction chromatography for proteins.

### Example 6: Purification of biomolecules at a larger scale

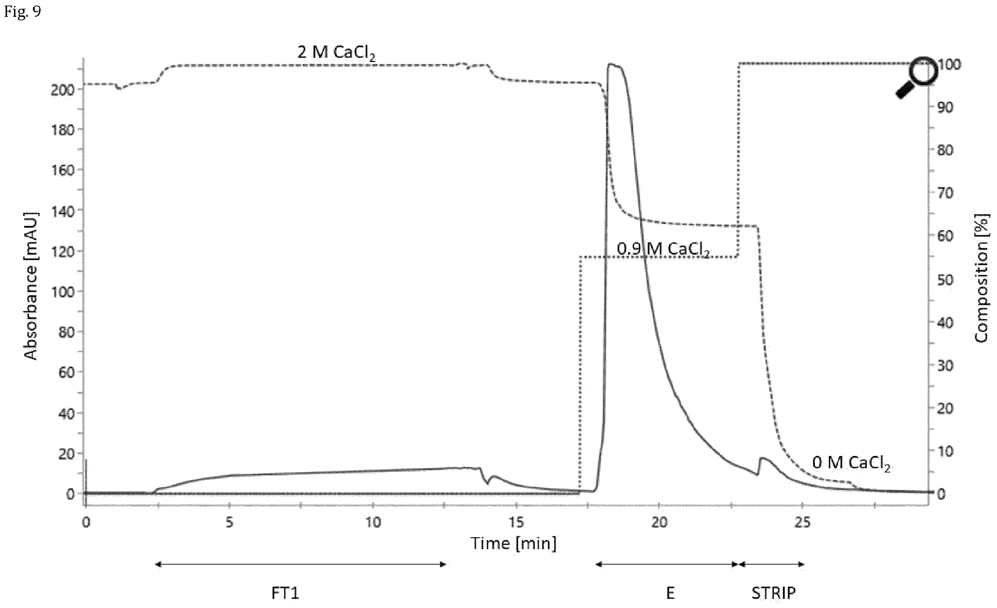

This example represents a scale-up experiment, performed the same way and with the same buffers as experiments in Example 1 but on a larger chromatography support. Plasmid DNA sample comprising 2 M CaClz at pH 7.2 was loaded onto CIMmultus^{®} C4 HLD (40 mL, 2 µm channel diameter, Sartorius BIA Separations) until the second pDNA breakthrough event occurred (which indicates that the column capacity was exceeded). The chromatography support had a 40 times larger volume than the one used in Example 1. A preparative chromatogram is shown in Fig. 9. Plotted are the UV absorbance value (continuous line), the salt concentration (dotted line) and the conductivity (dashed line).

Looking to Fig. 9, the sample was loaded onto the support between the 2-minute and 13.5-minute timestamps, the support was washed approx. between the 13.5-minute and 17-minute timestamps, the first solution was passed through the column approx. between the 17-minute and 23-minute timestamps, and the wash solution was applied approx. between the 23-minute and 29-minute timestamps.

Fig. 9 shows that two breakthroughs occurred, which is indicated by the rapid increase of absorbance starting between the 2-minute and 2.5-minute timestamps (corresponding to OC pDNA which did not bind to the column) and at approx. the 11-minute timestamp (when the column capacity for SC pDNA was likely exceeded). Plasmid DNA was eluted from the support by applying the first solution, which is indicated by the large increase in the absorbance between approx. the 17.5-minute and 22.5-minute timestamps. More pDNA was removed from the support by applying the wash solution, which is indicated by a smaller absorbance peak around the 24-minute timestamp.

HPLC analytics (data not shown) indicated that the load fraction contained both sc pDNA (82 %) and oc pDNA, whereas the FT1 fraction contained >99 % oc pDNA and the E fraction contained >95 % sc pDNA. The binding capacity observed in this experiment (1.95 mg/mL) was comparable to the one obtained with 1 mL column (1.85 mg/mL). With this example it was confirmed that using CaClz as a mobile phase modifier in hydrophobic interaction chromatography for pDNA is a scalable approach.

## Claims

1. Use of at least one multivalent halide salt as mobile phase modifier in hydrophobic interaction chromatography.

2. Use according to claim 1, wherein the at least one multivalent halide salt comprises divalent and/or trivalent metal cations, preferably Mg, Ca, Sr, Al, Fe, and/or Ba, more preferably Mg and/or Ca cations.

3. Use according to claim 1 or 2, wherein the at least one multivalent halide salt comprises F anions, Cl anions, Br anions, I anions, or At anions, or any combination thereof, preferably F and/or Cl anions.

4. Use according to any one of the preceding claims, wherein the at least one multivalent halide salt is MgClz and/or CaClz.

5. Use according to any one of the preceding claims, wherein the at least one multivalent halide salt is present at a total maximum concentration of 0.25 M - 5.0 M.

6. Use according to any one of the preceding claims, wherein the at least one multivalent halide salt is present in at least one buffer having a pH value in the range from 2.5 - 11.

7. Use according to any one of the preceding claims, wherein the mobile phase comprises less than 0.5 mol/L kosmotropic salt, preferably less than 0.05 mol/L kosmotropic salt, more preferably wherein the mobile phase is essentially free from kosmotropic salt.

8. Use according to any one of the preceding claims for the purification of biomolecules, wherein preferably the biomolecules are selected from the group consisting of nucleic acids, for example plasmid DNA, polypeptides, viruses, and virus-like particles.

9. Method for purifying biomolecules using hydrophobic interaction chromatography, the method comprising:
- passing a sample comprising the biomolecules through the chromatography support, wherein the sample further comprises at least one multivalent halide salt at a first concentration, and
- passing a first solution through the chromatography support, wherein the first solution comprises the at least one multivalent halide salt at a second concentration,
wherein the second concentration is lower than the first concentration.

10. Method according to claim 9, wherein the biomolecule is a nucleic acid, preferably plasmid DNA, and/or a polypeptide.

11. Method according to claim 9 or 10, wherein the sample additionally comprises a buffer to maintain the pH in the range of 2.5 - 11.

12. Method according to any one of claims 9 to 11, wherein the at least one multivalent halide salt is present in the sample at a total concentration of 0.25 - 5.0 M.

13. Method according to any one of claims 9 to 12, wherein the at least one multivalent halide salt is present in the sample at a total concentration of 0.25 - 5.0 M,
wherein preferably, passing the first solution through the chromatography support comprises applying a decreasing salt gradient of the at least one halide salt, wherein preferably the concentration of the multivalent halide salt is decreased from an initial concentration of 0.25 - 5 M to a final concentration of 0 - 1 M, wherein preferably, the decrease is carried out in a continuous or step-wise fashion.

14. Method according to any one of claims 9 to 13, wherein the chromatography support is suitable for hydrophobic interaction chromatography or reverse-phase chromatography, preferably wherein the chromatography support comprises neutral ligands selected from the group consisting of alkyl, aryl, cycloalkyl, hydroxy, epoxy, ether, amide and ester, and/or multimodal hydrophobic-hydrogen bonding ligands such as pyridine, further preferably wherein the chromatography support is a porous particle support, a membrane support, a monolith support or a nanofiber support.

15. Use of a chromatography support in a method according to any one of claims 9 to 14.

16. Use according to claim 15, wherein the chromatography support comprises hydrophobic ligands selected from the group consisting of alkyl, aryl and cycloalkyl.

17. Kit for the purification of biomolecules comprising:
- a chromatography support comprising hydrophobic ligands, and
- at least one multivalent halide salt.

18. Kit according to claim 17, wherein the at least one multivalent halide salt is present in a buffer solution, preferably in the form of a stock solution.

19. Kit according to claim 17 or 18, wherein the hydrophobic ligands are selected from the group consisting of alkyl, aryl and cycloalkyl or any combinations thereof.
